# EUROPEAN PATENT SPECIFICATION

(11) **EP 4 110 312 B1**
(45) Date of publication and mention of the grant of the patent: **25.11.2026**
(21) Application number: 21712359.5
(22) Date of filing: 23.02.2021
(51) Int. Cl.: A61K 31/12, A61K 38/17, A61K 45/06, A61P 3/14, A61P 9/00, A61P 21/00, A61P 25/00

(54) **APOAEQUORIN AND CURCUMIN CONTAINING COMPOSITIONS AND METHODS**
APOAEQUORIN UND CURCUMIN ENTHALTENDE ZUSAMMENSETZUNGEN UND VERFAHREN
COMPOSITIONS CONTENANT DE L'APOAEQUORINE ET DE LA CURCUMINE ET MÉTHODES

(30) Priority: 24.02.2020 US 202062980785 P
(43) Date of publication of application: 04.01.2023
(73) Proprietor: Quincy Bioscience, LLC, Middleton, WI 53562 (US)
(72) Inventor: UNDERWOOD, Mark, Y., Coppell, Texas 75019 (US)
(74) Representative: J A Kemp LLP
(86) International application number: PCT/US2021/019231
(87) International publication number: WO 2021/173550

(56) References cited:
- WO-A1-2009/114597
- WO-A2-2015/159272
- MAHMOOD KASHIF ET AL: "Recent developments in curcumin and curcumin based polymeric materials for biomedical applications: A review", INTERNATIONAL JOURNAL OF BIOLOGICAL MACROMOLECULES, ELSEVIER BV, NL, vol. 81, 21 September 2015 (2015-09-21), pages 877 - 890, XP029349673, ISSN: 0141-8130, DOI: 10.1016/J.IJBIOMAC.2015.09.026
- NAGHAVI FARZANEH SADAT ET AL: "Post-stroke neuronal circuits and mental illnesses", vol. 11, no. 1, 1 January 2019 (2019-01-01), pages 1 - 11, XP093332657, ISSN: 1944-8171, Retrieved from the Internet <URL:https://pubmed.ncbi.nlm.nih.gov/30911356/>
- ESMAILY HABIBOLLAH ET AL: "An investigation of the effects of curcumin on anxiety and depression in obese individuals: A randomized controlled trial", CHINESE JOURNAL OF INTEGRATED MEDICINE /ZHONGGUO JIEHE YIXUE ZAZHI (ENGLISH), ZHONGGUO ZHONG-XIYI JIEHE YANJIUHUI, CN, vol. 21, no. 5, 17 March 2015 (2015-03-17), pages 332 - 338, XP035497173, ISSN: 1672-0415, [retrieved on 20150317], DOI: 10.1007/S11655-015-2160-Z
- SPENCE J ET AL: "A brief review of three common supplements used in Alzheimer's disease", CONSULTANT PHARMACIST, THE SOCIETY, ARLINGTON, VA, US, vol. 32, no. 7, 1 July 2017 (2017-07-01), pages 412 - 414, XP009527416, ISSN: 0888-5109, DOI: 10.4140/TCP.N.2017.412

## Description

This International application claims the benefit of U.S. Provisional Application No. 62/980,785, filed February 24, 2020.

### STATEMENT REGARDING FEDERALLY SPONSORED RESEARCH OR DEVELOPMENT

Not Applicable.

### FIELD OF THE INVENTION

This invention relates generally to compositions useful for the maintenance of human health. In particular, this invention is directed to specific apoaequorin and curcumin containing compositions for oral administration, for use in treating a mental disorder.

### SUMMARY OF THE INVENTION

For clarity, any references herein to methods of therapy involving administration of particular substances or compositions are to be interpreted as being directed to the relevant substance or composition for use in the relevant method of therapy.

The presently claimed invention provides various advantages over prior compositions for use in treating a mental disorder. Such compositions include (a) an effective amount of apoaequorin, specifically approximately 10 mg/dose to 80 mg/dose; (b) an effective amount of curcumin, specfically approximately between 80 mg/dose to 1200 mg/dose; and (c) an acceptable carrier, for oral administration.

In certain embodiments, the compositions are administered to the subject in the form of nutraceutical compositions. In certain embodiments, nutraceutical compositions include, in addition to apoaequorin and curcumin, at least one other component recognized as providing nutraceutical benefit such as, for example, an immune boosting agent, anti-inflammatory agent, anti-oxidant agent, anti-viral agent, or a mixture thereof. Apoaequorin and curcumin compositions in certain embodiments are provided in a unit dosage form selected from a tablet, a capsule, a solution, a suspension, a syrup, or a beverage.

Disclosed herein is a method for treating a symptom or disorder associated with calcium imbalance, comprising administering to a subject in need of such treatment an effective amount of apoaequorin and curcumin (it being noted, however, that the presently claimed invention is directed, specifically, to a composition for use in treating a mental disorder).

Methods according to the disclosure are useful in treating a wide variety of symptoms or disorders associated with human health, including but not limited to mental disorder, anxiety, cognitive function, sleep quality, energy quality, mood quality, pain, memory quality. In certain aspects of the disclosure, the symptom of disorder is physiologically-related to neuronal excitability, muscle contraction, membrane permeability, cell division, hormone secretion, bone mineralization, or cell death following ischemia. In such methods, apoaequorin and curcumin are preferably administered to the subject in the form of a nutraceutical composition.

In yet another aspect of the disclosure, the disclosure encompasses the use of apoaequorin and curcumin for the manufacture of a nutraceutical composition for treating a symptom or disorder associated with human health in a subject administered the nutraceutical composition. Exemplary symptoms or disorders treated by such compositions include those associated with mental disorder, anxiety, cognitive function, sleep, energy, mood, pain, or memory.

Accordingly, the present disclosure further contemplates apoaequorin and curcumin for use in treating a symptom or disorder described above in a subject, including those symptoms or disorders associated with, e.g., mental disorder, anxiety, cognitive function, sleep, energy, mood, pain, or memory in a subject.

The present disclosure provides various advantages over prior compositions and methods in that it provides for the general improvement of a subject's mental and physical health.

Other objects, features and advantages of the present invention will become apparent after review of the specification and claims.

### BRIEF DESCRIPTION OF THE DRAWINGS

Figure 1 provides a graph showing the percent change from baseline of scores from areas: overall quality of sleep, energy, mood, pain and general heath vs. days 0 through 90.
Figure 2 depicts a graph showing data in which apoaequorin (10mg) was taken daily by 56 participants. The participants were evaluated from eight days to 30 days. The memory study showed a statistically significant improvement in memory after 30 days (hp<.05). 57% of participants had improvement in general memory, 51% in retaining information, 84% in remembering driving directions and 66% in word recall. N=56; 66% female, 34% male, mean age = 56 years; range 20-78 years.
Figure 3 provides a graph showing the percent change, from baseline, of scores from standardized cognitive battery questionnaire vs. day 0 through 90.

### DETAILED DESCRIPTION OF THE INVENTION

### IN GENERAL

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described.

### THE INVENTION

Aequorin is a photo-protein originally isolated from luminescent jellyfish and other marine organisms. The aequorin complex comprises a 22,285-dalton apoaequorin protein, molecular oxygen and the luminophore coelenterazine. When three Ca²⁺ ions bind to this complex, coelenterazine is oxidized to coelentermide, with a concomitant release of carbon dioxide and blue light. Aequorin is not exported or secreted by cells, nor is it compartmentalized or sequestered within cells. Accordingly, aequorin measurements have been used to detect Ca²⁺ changes that occur over relatively long periods. In several experimental systems, aequorin's luminescence was detectable many hours to days after cell loading. It is further known that aequorin also does not disrupt cell functions or embryo development.

Because of its Ca^{lf}-dependent luminescence, the aequorin complex has been extensively used as an intracellular Ca²⁺ indicator. *Aequorea victoria* aequorin has been specifically used to: (1) analyze the secretion response of single adrenal chromaffin cells to nicotinic cholinergic agonists; (2) clarify the role of Ca²⁺ release in heart muscle damage; (3) demonstrate the massive release of Ca²⁺ during fertilization; (4) study the regulation of the sarcoplasmic reticulum Ca²⁺ pump expression in developing chick myoblasts; and (5) calibrate micropipets with injection volumes of as little as three picoliters.

Apoaequorin has an approximate molecular weight of 22 kDa. Apoaequorin can be used to regenerate aequorin by reducing the disulfide bond in apoaequorin. The calcium-loaded apoaequorin retains the same compact scaffold and overall folding pattern as unreacted photoproteins containing a bound substrate.

Conventional purification of aequorin from the jellyfish *Aequorea victoria* requires laborious extraction procedures and sometimes yields preparations that are substantially heterogeneous or that are toxic to the organisms under study. Two tons of jellyfish typically yield approximately 125mg of the purified photoprotein. In contrast, recombinant aequorin is preferably produced by purifying apoaequorin from genetically engineered *Eseherichia coli,* followed by reconstitution of the aequorin complex *in vitro* with pure coelenterazine. Apoaequorin useful in the present invention has been described and is commercially-obtainable through purification schemes and/or syntheses known to those of skill in the art. S. Inouye, S. Zenno, Y. Sakaki, and F. Tsuji. High level expression and purification of apoaequorin. (1991) Protein Expression and Purification 2, 122-126.

WO 2009/114597 describes compositions containing apoaequorin and methods for their use in treating symptoms and disorders related to calcium imbalances associated with, for example, sleep quality, energy quality, mood quality, memory quality or pain.

Disclosed herein is the administration of apoaequorin and curcumin containing compositions to a subject in order to correct or maintain the calcium balance in that subject thereby improving, but not limited to, mental disorder, anxiety, cognitive function, sleep quality, energy quality, mood quality, pain, memory quality (again, it being noted for completeness that the presently claimed invention is directed, specifically, to a composition for use in treating a mental disorder). Curcumin (diferuloylmethane; 1,7-bis[4-hydroxy-3-methoxyphenyl]-1,6-heptadiene-3,5-dione) is a liposoluble molecule and a hydrophobic polyphenol derivative. Curcumin's known identifier is CAS Number:458-37-7. Its chemical formula is C21H20O6 with a molar mass of 368.39 g·mol-1 and melting point of 183° C. (361° F.; 456 K).

Curcumin and its mono- and dimethoxy derivatives known as curcuminoids, bisdemethoxy curcumin, demethoxy curcumin, and tetrahydro curcumin, are major constituents of the curcumin formulation derived from the extraction process. Commercial curcumin can sometimes contain approximately, 77% diferuloylmethane, 17% demethoxycurcumin, and 6% bisdemethoxycurcumin. Curcumin is obtained by extraction with solvent from the dried and ground rhizome of the Curcuma longa (turmeric) plant. Turmeric was historically used in Indian Ayurvedic medicine to treat a wide variety of disorders, but it was not until the 20th century that scientific research identified curcumin as the factor responsible for most of its biological activity.

Curcumin has formed the subject of numerous preclinical and clinical studies, so its anti-inflammatory and antioxidant power is well documented: it possesses the ability to regulate the oxidative balance of the cells, intervening in numerous mechanisms in different ways; in particular, it inhibits a series of factors with strongly inflammatory activity (COX 1 and 2, TNF, lipoxygenase and interferon-gamma). Finally, its efficacy as an antitumoral has been demonstrated in vitro (Bengmark S., JPEN, 2006, 30(1):45-51).

The curcumin (with the derivatives thereof, such as its esters which act as prodrugs) can be acquired as such (generally associated with other curcuminoids present in smaller proportions, such as Curcumin Complex), or as Biocurcumin (BCM-95^{®}) associated with oils and polymers that increase its bioavailability (US20070148263), or formulated in combination with piperine (Shobha et al., Planta Med, 1998, 64: 353-56), or obtained as micro- or nanoemulsions/nanodispersions that use lecithin and/or fatty acids and/or triglycerides and optionally also surfactants, such as Tween 80, to stabilize the molecule and make it more bioavailable.

It is also to be appreciated that diarylheptanoids are considered a class of compounds to which curcuminoids (e.g. curcumin) belongs. Other similar diarylheptanoids, such as those that may be obtained from ginger may possess similar properties as curcuminoids (e.g. curcumin). It will be appreciated that while curcuminoids (e.g. curcumin) may be described in detail herein, it will be appreciated that other diarylheptanoids may have similar or the same biological properties and effects, and which may be included in such compositions as described herein and may be used in the methods of treatment as described herein.

Furthermore, there is an increasing interest in the development of therapeutic compounds that may be used to improve focus, learning, memory and alertness, in both elderly and young people, individuals who need especially high memory and attention in their daily work, including students, construction workers, drivers, pilots, physicians, salespeople, executives, housewives, "high performance professionals" and people who are under mental or daily stress as well as persons who are prone to psychiatric instability and dramatic mood swings. A therapeutic product that can provide a balanced mental state would contribute to a more productive life for a large percentage of people who live a high stress, high performance life.

Thus, disclosed herein are methods whichcomprise administering apoaequorin and curcumin as the active ingredients for improving attention span. Additionally disclosed herein are methods which comprise administering apoaequorin and curcumin containing compositions in combination with one or more additional agents having known therapeutic or nutraceutical value.

As used herein, the terms "vigilance," "sustained attention," and "attention span" refer to an individual's ability to attend to a stimulus or object over a period of time. In other words, the ability to maintain focus and alertness over a period of time.

As used herein, the term "treating" includes preventative as well as disorder remittent treatment. As used herein, the terms "reducing", "alleviating", "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission.

As used herein, the term "administering" refers to bringing a patient, tissue, organ or cell in contact with apoaequorin and curcumin. As used herein, administration can be accomplished *in vitro,* i.e., in a test tube, or *in vivo,* i.e., in cells or tissues of living organisms, for example, humans. In preferred embodiments, the present invention encompasses orally administering the formulations or compositions useful in the present invention to a patient or subject. A "patient" or "subject", used equivalently herein, refers to a mammal, preferably a human, that either: (1) has a calcium imbalance-related disorder remediable or treatable by administration of apoaequorin and curcumin; or (2) is susceptible to a calcium imbalance-related disorder that is preventable by administering apoaequorin and curcumin.

As used herein, the terms "effective amount" and "therapeutically effective amount" refer to the quantity of active agents sufficient to yield a desired therapeutic response without undue adverse side effects such as toxicity, irritation, or allergic response. The specific "effective amount" will, obviously, vary with such factors as the particular condition being treated, the physical condition of the patient, the type of animal being treated, the duration of the treatment, the nature of concurrent therapy (if any), and the specific formulations employed and the structure of the compounds or its derivatives. In this disclosure, an amount would be deemed therapeutically effective if it resulted in one or more of the following: the prevention, the reversal, the stabilization, or the improvement in mental disorder, anxiety, cognitive function, sleep quality, energy quality, mood quality, pain, memory quality in a subject as compared to a subject not administered the combination. In certain aspects of the disclosure, the symptom of disorder is physiologically-related to neuronal excitability, muscle contraction, membrane permeability, cell division, hormone secretion, bone mineralization, or cell death following ischemia. The optimum effective amounts can be readily determined by one of ordinary skill in the art using routine experimentation.

In the presently claimed invention, in the compositions for oral administration to subjects, apoaequorin is formulated in a formulation at a dosage of approximately 10 mg/dose to 80 mg/dose, preferably 20 mg/dose to 70 mg/dose, and more preferably 30 mg/dose to 60 mg/dose. Curcumin, on the other hand, is formulated in a formulation at a dosage approximately between 80 mg/dose to 1200 mg/dose, with a preferable dosage for a subject approximately 600 mg/dose to 800 mg/dose.

Conventional excipients such as diluents, ligands, bufferings, sweeteners, flavorings, colorings, solubilizers, disintegrants, wetting agents and other excipients of common use may be added to the formulation. Suitable flavoring agents include natural flavors, artificial flavors, and mints, such as peppermint, menthol, artificial vanilla, cinnamon, various fruit flavors, both individual and mixed, and the like are contemplated. The flavorings are generally utilized in amounts that will vary depending upon the individual flavor, and may, for example, range in amounts of about 0.5 to about 3% by weight of the final composition weight.

In the instance where sweeteners are utilized, the present invention contemplates the inclusion of those sweeteners well known in the art, including both natural and artificial sweeteners. Thus, additional sweeteners may be chosen from the following non-limiting list: sugars such as sucrose, glucose (corn syrup), invert sugar, fructose, and mixtures thereof; saccharin and its various salts such as the sodium or calcium salt; cyclamic acid and its various salts such as the sodium salt; the dipeptide sweeteners such as aspartame; dihydrochalcone; glycyrrhizin; Stevia rebaudiana (Stevioside); and sugar alcohols such as sorbitol, sorbitol syrup, mannitol, xylitol, and the like. Also contemplated as an additional sweetener is the nonfermentable sugar substitute (hydrogenated starch hydrolysate) which is described in U.S. Pat. No. Re 26,959. Also contemplated is the synthetic sweetener 3,6-dihydro-6-methyl-1-1-2,3-oxathiazin-4-one-2,2-dioxide particularly the potassium (Acesulfame-K), sodium and calcium salts thereof as described in German Pat. No. 2,001,017.7. In general, the amount of sweetener will vary according to the type of sweetener and the desired taste of the final product. For example, natural sweeteners may be used in amounts up to about 5% by weight, while artificial sweeteners may be in amounts up to about 1% by weight.

The colorants useful in the present invention include pigments, such as titanium dioxide, that may be incorporated in amounts of up to about 1% by weight, and preferably up to about 0.6% by weight. Also, the colorants may include other dyes suitable for food, drug and cosmetic applications, and known as F.D. & C. dyes and the like. The materials acceptable for the foregoing spectrum of use are preferably water-soluble. Illustrative examples include indigoid dye, known as F.D. & C. Blue No. 2, which is the disodium salt of 5,5'-indigotin disulfonic acid. Similarly, the dye known as F.D. & C. Green No. 1, comprises a triphenylmethane dye and is the monosodium salt of 4-[4-N ethyl-p-sulfobenzylamino)diphenylmethylene][1-(N-ethyl-N-p-sulfoniumbenzyl)-2,5-cyclohexadienimine]. A full recitation of all F.D. & C. and D. & C. dyes and their corresponding chemical structures may be found in the Kirk-Othmer Encyclopedia of Chemical Technology, in Volume 5, at Pages 857-884.

Compositions may further include liquids or lyophilized or otherwise dried formulations and include diluents of various buffer content (e.g., Tris-HC1, acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polyglycolic acid, or hydrogels, or onto liposomes, microemulsions, micelles, lamellar or multilamellar vesicles, erythrocyte ghosts or spheroplasts. Such compositions will influence the physical state, solubility, stability, rate of *in vivo* release, and rate of *in vivo* clearance. Controlled or sustained release compositions include formulation in lipophilic depots (e.g., fatty acids, waxes, oils).

The preparation of compositions which contain an active component is well understood in the art. The active therapeutic ingredient is often mixed with excipients which are pharmaceutically acceptable and compatible with the active ingredient. Suitable excipients include, for example, water, saline, dextrose, glycerol, ethanol, or the like or any combination thereof. In addition, the composition can contain minor amounts of auxiliary substances such as wetting or emulsifying agents, pH buffering agents which enhance the effectiveness of the active ingredient.

An active component can be formulated into the composition as neutralized pharmaceutically acceptable salt forms. Pharmaceutically acceptable salts include the acid addition salts, which are formed with inorganic acids such as, for example, hydrochloric, or phosphoric acids, or such organic acids as acetic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Salts of active ingredients are preferably pharmaceutically acceptable salts. Other salts may, however, be useful in the preparation of the compositions according to the invention or of their pharmaceutically acceptable salts. Suitable pharmaceutically acceptable salts include acid addition salts which may, for example, be formed by mixing a solution of active ingredients with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid.

In addition, "supplement" for the purpose of this specification, refers to a food item, or a part of a food item, that offers medical health benefits, including prevention and/or treatment of disease. A supplement composition according to the present invention may contain only apoaequorin and curcumin as active ingredients, or alternatively, may further comprise, in admixture with dietary supplements including vitamins, co-enzymes, minerals, herbs, amino acids and the like which supplement the diet by increasing the total intake of that substance.

Disclosed herein are methods of providing health benefits to a patient comprising the step of administering to the subject a supplement composition containing apoaequorin and curcumin. Such compositions include an "acceptable carrier" which, as referred to herein, is any carrier suitable for oral delivery including aforementioned pharmaceutically-acceptable carriers suitable for the oral route. Thus, the disclosure provides a more favorable mechanism for orally delivering a medicament to a patient.

In certain embodiments, compositions according to the invention comprise dietary supplements which, defined on a functional basis, include immune boosting agents, anti-inflammatory agents, anti-oxidant agents, anti-viral agents, or mixtures thereof.

Immune boosters and/or anti-viral agents are useful for accelerating wound- healing and improved immune function; and they include extracts from the coneflowers, or herbs of the genus *Echinacea,* extracts from herbs of the genus *Sambuca,* and Goldenseal extracts. Herbs of the genus *Astragalus* are also effective immune boosters in either their natural or processed forms. *Astragalus* stimulates development of stem cells in the marrow and lymph tissue active immune cells. Zinc and its bioactive salts, such as zinc gluconate and zinc acetate, also act as immune boosters in the treatment of the common cold.

Antioxidants include the natural, sulfur-containing amino acid allicin, which acts to increase the level of antioxidant enzymes in the blood. Herbs or herbal extracts, such as garlic, which contain allicin, are also effective antioxidants. The catechins, and the extracts of herbs such as green tea containing catechins, are also effective antioxidants. Extracts of the genus *Astragalus* also show antioxidant activity. The bioflavonoids, such as quercetin, hesperidin, rutin, and mixtures thereof, are also effective as antioxidants. The primary beneficial role of the bioflavonoids may be in protecting vitamin C from oxidation in the body. This makes more vitamin C, or ascorbic acid, available for use by the body.

Bioflavonoids such as quercetin are also effective anti-inflammatory agents, and may be used as such in the inventive compositions. Anti-inflammatory herbal supplements and anti-inflammatory compounds derived from plants or herbs may also be used as anti-inflammatory agents in the inventive composition. These include bromolain, a proteolytic enzyme found in pineapple; teas and extracts of stinging nettle; turmeric, extracts of turmeric, or curcumin, a yellow pigment isolated from turmeric.

Another supplement which may be used in the present invention is ginger, derived from herbs of the genus *Zingiber.* This has been found to possess cardiotonic activity due to compounds such as gingerol and the related compound shogaol as well as providing benefits in the treatment of dizziness, and vestibular disorders. Ginger is also effective in the treatment of nausea and other stomach disorders.

Supplements which assist in rebuilding soft tissue structures, particularly in rebuilding cartilage, are useful in compositions for treating the pain of arthritis and other joint disorders. Glucosamine, glucosamine sulfate, chondroitin may be derived from a variety of sources such as Elk Velvet Antler. Marine lipid complexes, omega 3 fatty acid complexes, and fish oil are also known to be useful in treating pain associated with arthritis.

Supplements useful in treating migraine headaches include feverfew and *Gingko biloba.* The main active ingredient in feverfew is the sesquiterpene lactone parthenolide, which inhibits the secretions of prostaglandin which in turn cause pain through vasospastic activity in the blood vessels. Feverfew also exhibits anti-inflammatory properties. Fish oil, owing to its platelet-stabilizing and antivasospastic actions, may also be useful in treating migraine headaches. The herb *Gingko biloba* also assists in treatment of migraines by stabilizing arteries and improving blood circulation.

Although some of the supplements listed above have been described as to their pharmacological effects, other additives may also be utilized in the present invention and their effects are well documented in the scientific literature.

The invention will be more fully understood upon consideration of the following non-limiting Examples.

### III. REFERENCE EXAMPLES

Example 1: Administration of apoaequorin over a ninety (90) day time course results in improved quality of life for test subjects.

The present analysis, an open-label study, of 32 patients over a 90 day period shows an increase in overall quality of sleep, energy, mood, pain, general health. Changes in performance were measured via a standardized battery of questions. These included assessments of qualitative cognitive test, a sleep index, a headache index and a Quality of Life questionnaire. The study shows improved performance. No participants discontinued the study due to an adverse event.

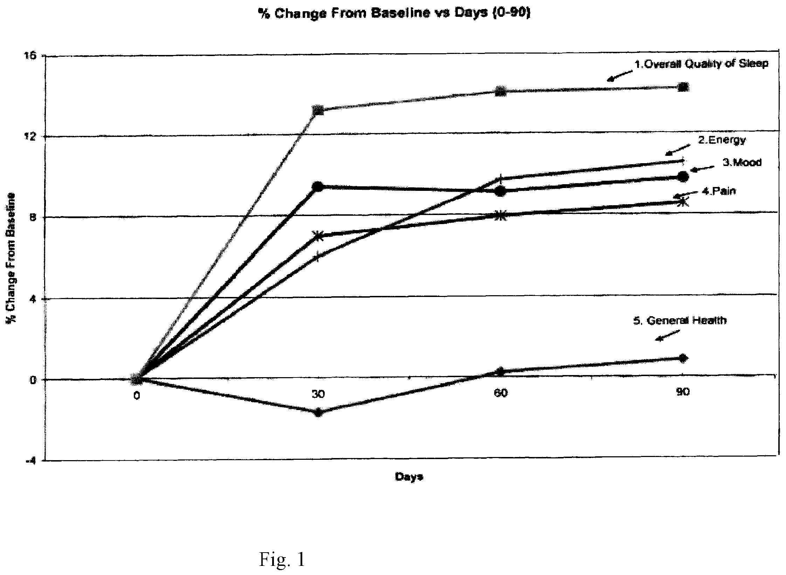

The results illustrated in Fig. 1 show the percent change from baseline of scores from the areas mentioned; we have excluded the memory scores for another graph. The analysis here is shown as marked on the graph as 1, 2, 3, 4 and 5 vs. days 0 through 90. The graph shows an increase in overall quality of sleep, energy, mood, pain and general health. The baseline was known from a pre-study phase.

Example 2: Administration of apoaequorin over a thirty (30) day time course results in improved quality of life for test subjects.

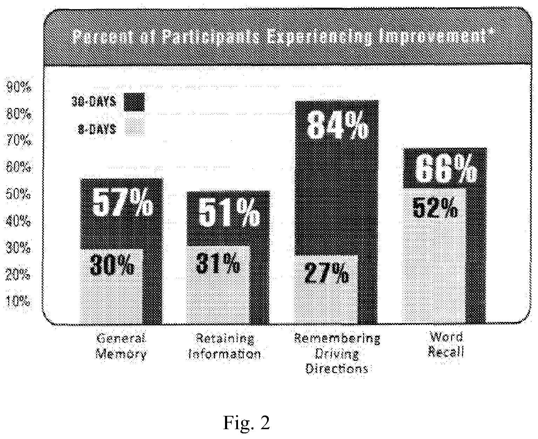

The present study was an open-label study for 56 participants over a 30 day period. Changes in performance were measured via a memory screening tool. As illustrated in Fig. 2, the study showed improved memory performance as early as eight days but with statistically greater improvement at day 30. No participants discontinued the study due to an adverse event.

Example 3: Administration of apoaequorin over a ninety (90) day time course results in improved cognition for test subjects.

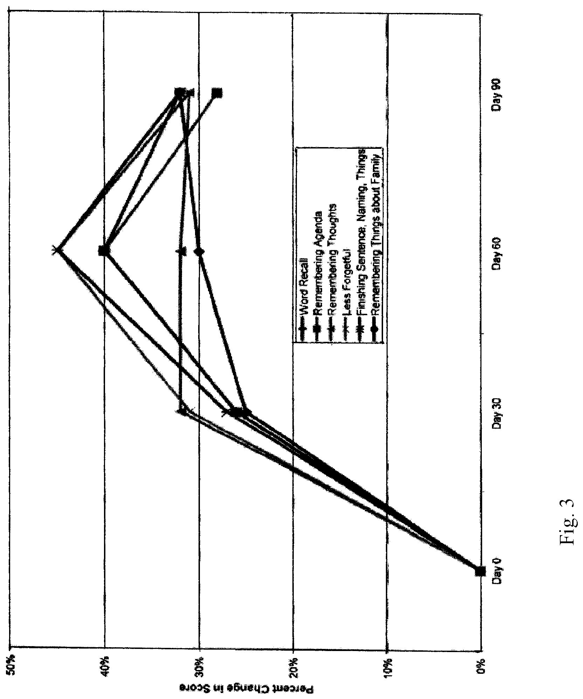

The present analysis, for an open-label study of 32 patients shows an increase in cognitive ability. Changes in performance were measured via a standardized cognitive battery. The study showed improved cognition as early as eight days but with statistically greater improvement at day 30, as well as 60-90. No participants discontinued the study due to an adverse event. The results shown in Fig. 3 demonstrate the significant percent increase from baseline of scores in cognitive ability. Note: Greater than 51% of participants had an increase in cognitive ability.

## Claims

1. A composition for oral administration, for use in treating a mental disorder, wherein said use comprises administering said composition to a subject in need thereof;
wherein said composition comprises: (a) an effective amount of apoaequorin; (b) an effective amount of curcumin; and (c) an acceptable carrier; and wherein the effective amount of apoaequorin is approximately 10 mg/dose to 80 mg/dose and the effective amount of curcumin is approximately between 80 mg/dose to 1200 mg/dose.

2. A composition for use according to claim 1, wherein said composition further comprises an immune boosting agent, anti-inflammatory agent, anti-oxidant agent, anti-viral agent, or a mixture thereof.

3. A composition for use according to claim 1 or 2, wherein said composition is in a unit dosage form selected from a tablet, a capsule, a solution, a suspension, a syrup, or a beverage.

4. A composition for use according to any one of claims 1 to 3, wherein said composition is in the form of a nutraceutical composition.

5. A composition for use according to any one of claims 1 to 4, wherein the composition is administered to said subject in the form of a nutraceutical composition.

6. A composition for use according to any one of claims 1 to 5, wherein said use comprises orally administering said composition to said subject.

## Patentansprüche

1. Zusammensetzung zur oralen Verabreichung zur Verwendung bei der Behandlung einer psychischen Störung, wobei die Verwendung die Verabreichung der Zusammensetzung an einen Patienten umfasst, der diese benötigt;
wobei die Zusammensetzung umfasst: (a) eine wirksame Menge an Apoaequorin; (b) eine wirksame Menge an Curcumin; und (c) einen annehmbaren Träger; und wobei die wirksame Menge an Apoaequorin etwa 10 mg/Dosis bis 80 mg/Dosis beträgt und die wirksame Menge an Curcumin etwa zwischen 80 mg/Dosis und 1200 mg/Dosis beträgt.

2. Zusammensetzung zur Verwendung nach Anspruch 1, wobei die Zusammensetzung ferner ein immunstärkendes Mittel, ein entzündungshemmendes Mittel, ein Antioxidans, ein antivirales Mittel oder ein Gemisch davon umfasst.

3. Zusammensetzung zur Verwendung nach Anspruch 1 oder 2, wobei die Zusammensetzung in einer Einzeldosisform vorliegt, die aus einer Tablette, einer Kapsel, einer Lösung, einer Suspension, einem Sirup oder einem Getränk ausgewählt ist.

4. Zusammensetzung zur Verwendung nach einem der Ansprüche 1 bis 3, wobei die Zusammensetzung in Form einer nutrazeutischen Zusammensetzung vorliegt.

5. Zusammensetzung zur Verwendung nach einem der Ansprüche 1 bis 4, wobei die Zusammensetzung dem Patienten in Form einer nutrazeutischen Zusammensetzung verabreicht wird.

6. Zusammensetzung zur Verwendung nach einem der Ansprüche 1 bis 5, wobei die Verwendung die orale Verabreichung der Zusammensetzung an den Patienten umfasst.

## Revendications

1. Composition destinée à une administration par voie orale, destinée à être utilisée dans le traitement d'un trouble mental, ladite utilisation comprenant l'administration de ladite composition à un sujet qui en a besoin,
ladite composition comprenant : (a) une quantité efficace d'apoaéquorine, (b) une quantité efficace de curcumine et (c) un excipient acceptable ; la quantité efficace d'apoaéquorine étant d'environ 10 mg/dose à 80 mg/dose et la quantité efficace de curcumine étant d'environ 80 mg/dose à 1200 mg/dose.

2. Composition destinée à être utilisée selon la revendication 1, ladite composition comprenant en outre un agent stimulant le système immunitaire, un agent anti-inflammatoire, un agent antioxydant, un agent antiviral ou un mélange de ceux-ci.

3. Composition destinée à être utilisée selon la revendication 1 ou 2, ladite composition se présentant sous une forme posologique unitaire sélectionnée parmi un comprimé, une gélule, une solution, une suspension, un sirop ou une boisson.

4. Composition destinée à être utilisée selon l'une quelconque des revendications 1 à 3, ladite composition se présentant sous la forme d'une composition nutraceutique.

5. Composition destinée à être utilisée selon l'une quelconque des revendications 1 à 4, ladite composition étant administrée audit sujet sous la forme d'une composition nutraceutique.

6. Composition destinée à être utilisée selon l'une quelconque des revendications 1 à 5, ladite utilisation comprenant l'administration par voie orale de ladite composition audit sujet.
